Europäisches Patentamt

European Patent Office

Office européen des brevets

(19)

(11) **EP 0 674 492 B1**

(12) **EUROPÄISCHE PATENTSCHRIFT**

(45) Veröffentlichungstag und Bekanntmachung des
Hinweises auf die Patenterteilung:
**15.07.1998 Patentblatt 1998/29**

(21) Anmeldenummer: **94901755.2**

(22) Anmeldetag: **14.12.1993**

(51) Int. Cl.$^6$: **A61B 5/00**

(86) Internationale Anmeldenummer:
**PCT/DE93/01193**

(87) Internationale Veröffentlichungsnummer:
**WO 94/13193 (23.06.1994 Gazette 1994/14)**

(54) **VERFAHREN UND VORRICHTUNG ZUR NICHTINVASIVEN KONZENTRATIONSBESTIMMUNG POLARISIERENDER STOFFE**

METHOD AND DEVICE FOR NON-INVASIVELY DETERMINING THE CONCENTRATION OF POLARISING SUBSTANCES

METHODE ET DISPOSITIF SERVANT A DETERMINER DE MANIERE NON INVASIVE LA CONCENTRATION EN SUBSTANCES POLARISANTES

(84) Benannte Vertragsstaaten:
**CH DE FR GB IT LI SE**

(30) Priorität: **15.12.1992 DE 4242232**

(43) Veröffentlichungstag der Anmeldung:
**04.10.1995 Patentblatt 1995/40**

(73) Patentinhaber: **KUHLS, Burkhard**
**D-80687 München (DE)**

(72) Erfinder: **KUHLS, Burkhard**
**D-80687 München (DE)**

(74) Vertreter:
**Haft, von Puttkamer, Berngruber, Czybulka**
**Patentanwälte**
**Franziskanerstrasse 38**
**81669 München (DE)**

(56) Entgegenhaltungen:
**EP-A- 0 282 234        WO-A-90/07905**
**WO-A-92/07511        US-A- 3 877 816**
**US-A- 5 009 230**

- **PATENT ABSTRACTS OF JAPAN vol. 13, no. 120 (P-846) 24. März 1989 & JP,A,63 293 442 (SUMITOMO METAL IND. LTD.) 30. November 1988**

**Beschreibung**

Die Erfindung betrifft eine Vorrichtung zur nichtinvasiven Messung polarisierender Stoffe im menschlichen Blut, insbesondere zur Messung der Blutzuckerkonzentration, gemäß dem Oberbegriff des Patentanspruches 1.

Bei zuviel Zucker im menschlichen Blut wird Insulin aus der Bauchspeicheldrüse (Pankreas) freigesetzt. Dieses Insulin verteilt sich im Blut und veranlaßt die Zellmembranen sämtlicher erreichbarer Zellen dazu, daß sie leitfähiger für die Glucose- Aufnahme werden. Hierdurch sinkt der Blutzuckergehalt, da die Zellen den Zucker aufnehmen und verwerten (Glykolyse zur Zellversorgung; Glycogensynthese in der Leber). Diabetiker haben dauerhaft erhöhte Blutzuckerwerte (diabetes mellitus) bedingt durch Insulinmangel. Dies ist eine der häufigsten Zivilisationskrankeiten; die Blutzuckerkonzentration muß ständig kontrolliert und medikamentös beeinflußt werden, damit nicht irreparable Langzeitschäden auftreten.

Neben der Bestimmung der Blutzuckerkonzentration durch Entnahme einer kleinen Blutmenge und Aufbringen etwa auf einen kleinen Teststreifen oder der Bestimmung der Blutzuckerkonzentrationim Harn mit Hilfe eines Polarimeters sind auch nichtinvasive Verfahren bekannt, bei deren die Tatsache ausgenutzt wird, dass Blutzucker optisch aktiv ist; vgl etwa die WO 92/07511, von der die vorliegende Erfindung ausgeht, oder die US-A-5009230.

Bei der Vorrichtung gemäss der WO 92/07511 wird das Licht einer Lichtquelle mit einem Polarisator linear polarisiert und der Polarisator rotierend angetrieben. In einem Strahlteiler wird der polarisierte Lichtstrahl aufgeteilt in einen Referenz- und einen Messstrahl. Der Messstrahl wird durch die Hornhaut des menschlichen Auges und das Kammerwasser im Augeninneren geleitet, wonach anschliessend die Verdrehung der Polarisationsebene des Messstrahles gegenüber derjenigen des Referenzstrahles und daraus und aus der Schichtdicke d der durchstrahlten Lösung die Blutzuckerkonzentration bestimmt wird.

Hierbei wird von der Tatsache Gebrauch gemacht, dass die Zuckerkonzentration im Kammerwasser höher als im Blut, der Blutzuckerkonzentration jedoch im wesentlichen proportional ist. Da jedoch die Zuckerkonzentrationen im Blut und im Kammerwasser des Auges gering sind, muss ein hoher apparativer Aufwand getrieben werden, um brauchbare Messergebnissse zu erhalten. Dies wird bei der bekannten Vorrichtung dadurch versucht, dass das Licht der Lichtquelle durch drei Polarisationseinrichtungen geführt wird, in denen es erst linear, dann zirkular und anschliessend wiederum in einem rotierenden Polarisator linear polarisiert wird. Auf diese Weise erhält man eine Polarisation mit einer zeitlich konstanten Amplitude.

Das Polarimeter gemäß der US-PS 5,009,230 wird definiert zwischen zwei Analysatorstellungen umgeschaltet. Durch Differenzbildung der erfaßten Meßwerte wird eine dem Differenzwinkel entsprechende Intensitätsänderung gemessen, die der Glukosekonzentration proportional ist. Diese Messung kann nichtinvasiv bei verschiedenen Wellenlängen als Transmissionsmessung vorgenommen werden.

Diese Vorrichtung hat jedoch folgende Nachteile :Durch Umschalten sind nach 'Fourier' im einem solchen 'Rechteck-Impuls' theoretisch unendlich viele Oberwellen im Meßsignal vorhanden, so daß sich die Energie, besonders bei schnellen Schaltfolgen über das gesamte Spektrum verteilt. Bei einem derart kleinem Polarisationswinkel, wie er vom Blut hervorgerufen wird, ist das Meßsignal durch Rauschen überdeckt. Da die Rauschleistung abhängig ist von der Bandbreite der zugelassenen Spektren, ist der Meßwert hiermit kaum zu identifizieren. Ferner ist die Meßvorrichtung beschränkt auf eine Transmissionsmessung, so daß nur direkt durchstrahlbare Körperteile dazu in Frage kommen.

Der Erfindung liegt die Aufgabe zugrunde, eine Vorrichtung zum Bestimmen der Blutzuckerkonzentration im Blut anzugeben, mit der eine nichtinvasive quasikontinuierliche Bestimmung mit hoher Meßgenauigkeit möglich ist.

Diese Aufgabe ist gemäß der Erfindung durch die Merkmale des Patentanspruches 1 gelöst.

Weitere Ausgestaltungen der Erfindung gehen aus den Unteransprüchen hervor.

Mit der Erfindung wird ferner ein einfaches Verfahren zum Auswerten der Meßsignale angegeben, das besonders günstig mit der erfindungsgemäßen Vorrichtung ausgeführt werden kann.

Blutzucker ist ein Monosacharid und wird als D(+)- Glucose bezeichnet; vgl. Linear polarisiertes Licht wird durch gelösten Blutzucker aufgrund der optischen Aktivität aus seiner ursprünglichen Polarisationsrichtung nach rechts (Uhrzeigersinn) gedreht. Bei Lösungen optisch aktiver Substanzen ist der Drehwinkel $\alpha$ der Konzentration C proportional., und es gilt:

$$\alpha = \alpha_0 * C * d \qquad\qquad (1)$$

Worin bedeuten:

C      :Konzentration des optisch aktiven Stoffes (hier: Blutzucker )

$\alpha$      :Drehwinkel der Polarisationsrichtung abhängig von der jeweiligen Schichtdicke

$\alpha_0$     :spezifische Drehung; (möglist in Blut bestimmen)

d :drehende Schichtdicke

Die erfindungsgemäße Vorrichtung bewirkt, daß die Blutzuckerkonzentration über Polarisation und Extinktion im dynamisch pulsierenden Blut nichtinvasiv bei geringer Polarisation kontinuierlich bestimmt wird. Hierbei wird eine hohe Genauigkeit der Meßvorrichtung erreicht. Geht man von einer durchschnittlich 2mm dicken Blutschichtdicke z.B. im Finger aus, so dreht sich die Polarisationsebene (Rohrzucker: $\alpha_0$= 66,5°/dm) um ca. 0.013° bei einem normalen Blutzuckerwert von 1g/l (100mg/dl). D.h. bei einer Meßgenauigkeit von 10% ist eine Genauigkeit von 1/1000° erforderlich. Der periodisch rotierende Polarisator wandelt den Drehwinkel der Polarisation in einen Phasenwinkel des der Phasenverschiebung durch das Meßobjekt zur Referenzphase sowie über eine statische Extinktionsmessung (Gewebe und Blut) und eine dynamische Messung (nur pulsativer Anteil des Blutes) läßt sich die Blutzuckerkonzentration nichtinvasiv bestimmen Die Messung ist als Transmissionsmessung oder als Streumessung möglich.

Bei der Vorrichtung wird polarisiertes Licht in das Meßobjekt (z.B. Finger, Ohrläppchen...).eingekoppelt. Die Auskopplung erfolgt über einen mit der Frequenz f rotierenden Analysator (Bild 2) oder durch Anwendung der Magnetorotation (z.B.:Faraday-Effekt) über ein mit dr Frequenz $\Omega$ schwingendes Magnetfeld. Der Analysator wandelt den Drehwinkel der Schwingungsrichtung in einen Phasenwinkel des Intensitätsverlaufes des durchgelassenen Lichtes. Die Reihenfolge Analysator/Meßobjekt ist unerheblich für die Messung.

Hierbei wird das polarisierte Licht durch die periodische Krafteinwirkung am Analysator/ Polarisator z.B. sinusförmig moduliert. Zur periodischen Polarisationsdrehung (Modulation ) des Analysators bzw Polarisators bestehen z.B. folgende Möglichkeiten: Über mechanische Drehung (Rotationsantrieb) wird Polarisationsrichtung verändert:

- Polarisationsfolien (Film durchsichtiger Kunststoffe mit Miszellen; vgl. Pohl: Optik und Atomphysik 13.Aufl. S.116.

- Zellulosehydratfolien (durch Streckung wird eine gerichtete Spannungsdoppelbrechung hervorgerufen; vgl. Bergmann/Schaefer:Lehrbuch der Experimentalphysik Optik Bd.III 7.Aufl. S.515.

- Über ein magnetisches Feld wird diePolarisationsrichtung verändert:

  - Magnetorotation (Faraday- Effekt): durch ein Magnetfeld wird die Polarisatinsrichtung gedreht (z.B.: Bleisilikatglas, Steinsalz,..)[1]

Über ein elektrisches Feld wird die Polarisationsrichtung periodisch verändert:

- Polarisationsrichtung wird durch Anlegen eines äußeren elektrischen Feldes gedreht

Für die vom Analysator durchgelassene Intensität z.B. bei sinusförmiger Modulation gilt:

$$I_{Analysator} = I_{max} * \sin^2(\Omega t - \alpha)$$

$I_{max}$ = Maximum der durchgelassenen Intensität oder nach Anwendung der Additionstheoreme:

$$I_{Analysator} = I_{max}/2 * [1 - \cos(2\Omega t - 2\alpha)]$$

Die Intensität des durchgelassenen Lichtes oszilliert demnach mit der doppelten Rotationsfrequenz $\omega$ (=2*$\Omega$) . Der Transimpedanz- Verstärker (TIV) liefert daher die Ausgangsspannung:

$$Ua(TIV) = Umax *[1 - \cos(\omega t - 2\alpha)]$$

wobei $U_{max}$ der maximalen Intensität $I_{max}$ entspricht. Der Polarisationswinkel $\alpha$ wird durch den optisch aktiven Blutzucker verdreht.

Streuung beeinflußt die vorherrschende Polarisationsrichtung nur insofern , daß die vorherrschende Polarisationsrichtung deterministisch abgebaut wird und somit das Signal schwieriger zu identifizieren ist.

Über einen Winkelgeber wird synchron dazu die Frequenz und Phasenlage als Referenz gemessen. Mit Hilfe dieser Referenzmessung kann das eigentliche Signal, welches vom Meßobjekt herrührt ,verglichen und ausgewertet werden. Der Winkelgeber kann elektrisch, optisch oder magnetisch arbeiten.

Als polarisierte Lichtquelle bieten sich Halbleiter-Laserdioden an. Oberhalb des Schwellstromes emittieren Laserdioden linearpolarisierces Licht und zwar mit einer Polarisationsebene, die parallel zum pn- Übergang liegt. Da jedoch ein Teil der Strahlung durch spontane Emission entsteht, ist die Polarisationsreinheit nie hundertprozentig. Mit zunehmender Laserleistung steigt auch dieser Wert[2]. Bei der oben angewendeten Vorrichtung reicht ein überwiegend polarisiertes Licht zur Detektion bereits aus. Völlig polarisiertes Licht als Lichtquelle über Polarisator ( z.B. mit Folie oder Brewester- Fenster) könnte den Signal-Rauschabstand allerdings erhöhen.

Als Referenz wird ein Winkelgeber btw. ein Phasengeber verwendet, die das Referenzsignal liefern. Dies wird z. B. direkt am Ausgang des Motors, Polarisators oder Analysators gemessen oder vom Funktionsgenerator direktangegeben. Ein Detektor erfaßt hierbei optisch (reflex oder transparent ), magnetisch , elektrisch oder elektrostatisch die momentane Phasenlage.

Zur Auswertung wird sowohl die Polarisation als auch die Extinktion herangezogen. Um nur das pulsierende Blut selbst nichtinvasiv messen zu können, wird der Umstand benutzt, daß das bei einer dynamischen Messung (nur Blutpulsation) maßgebliche Blut selbst registriert wird.

Zur Extinktionsmessung wird mit monochromatischem Licht gemessen. Die Extinktion bei den Lichtwellenlängen zwischen 600nm bis 780nm kann stark schwanken, da sie hier von der momentanen Sauerstoffsättigung im Blut abhängt. Um unabhängig von der Sauerstoffsättigung im Blut zu messen, sind Lichtwellenlängen an den 'isobestischen Punkten' um 550 nm und um 805 nm besonders geeignet.

Um die sicherheitstechnischen Anforderungen zu erfüllen, kann das Träger- Signal und die Information auch über polarisationserhaltende Lichtwellenleiter zum Meßobjekt hin und zurück geleitet werden. Wobei die rotierende Scheibe entweder direkt am Meßobjekt oder extern das polarisierte Licht moduliert.

Die Vorrichtung läßt sich auch in Subminiaturform anfertigen, wobei sie dann als Transplantat auch invasiv eingesetzt werden kann.

Die erhaltenen Meßwerte aus der oben beschriebenen Vorrichtung werden zur nichtinvasiven Glukose- Bestimmung über Elektronik oder per Programm nach folgendem Schema codiert:

Durch eine Kalibrations-Messung wird das Meßgerät bei bekannter Blutzuckerkonzentration C1 am späteren Meßobjekt eingeeicht. Es gilt

$\alpha$     :Winkel der Drehung der Polarisationsebene (hervorgerufen durch optisch aktive Substanz- hier: Glukose)
$C$     : Blutzuckerkonzentration $k_{pol}$ : Gewebepolarisation (Konstante; nicht von der Blutpulsation abhängig)
$d$     :effektiv wirkende Schichtdicke der Blutgefäße
$\Delta d$     : Pulshub (nur der dynamisch pulsierende Anteil der Blutgefäße)

$$\alpha = \alpha_0 * C * d + k_{pol} \qquad\qquad (8) \quad \text{Polarisationswinkel}$$

Um nur den im Blut vorhandenen Blutzucker zu erfassen, werden nur die durch den Blutpuls bewirkten dynamischen Winkeländerungen herangezogen.

Hierbei gilt fur kleine $\Delta d_{Gefäß}$:

$$\frac{\Delta \alpha}{\Delta d} = \frac{\partial \alpha}{\partial d} = \alpha_0 * C \qquad\qquad (10)$$

$$\Delta \alpha = \Delta d * \alpha_0 * C$$

wobei $k_{pol}$ verschwindet.

Nach Eineichung mit der Konzentration $C_1$ ergibt sich dann für eine unbekannte Konzentration $C_{meß}$:

$$\frac{\Delta \alpha_{meß}}{\Delta \alpha_1} = \frac{C_{meß} * \Delta d_{meß}}{C_1 * \Delta d_1}$$

bzw:

$$C_{meß} = C_1 * \frac{\Delta \alpha_{meß} * \Delta d_1}{\Delta \alpha_1 * \Delta d_{meß}} \qquad\qquad (12)$$

Darin kürzen sich die Pulshübe $\Delta d$ nur bei identischen Kreislaufwerten (Blutdruck, Puls, usw) vollständig heraus. Allgemein muß man jedoch das Verhältnis $\Delta d_1/\Delta d_{meß}$ als Funktion der einzelnen Kreislaufparameter z.B. in Form einer Eichkurve ermitteln. Dies läßt sich umgehen, wenn die Pulshübe durch eine zusätzliche Extinktions- Messung bestimmt werden.

Aus der Lambert- Beerschen Gleichung erhält man durch Differentiation nach der Schichtdicke d :

$I_{in}$     : eingestrahlte Intensität
$I_{meß}$     : gemessene Intensität
$C_{häm}$     : Hämatokritkonzentrtion; im Blut (Männer 510 g/l bzw 0.47 ml Zellen/dl Blut, Frauen 460 g/l bzw 0.42 ml Zellen/dl Blut)
d     :effektiv wirkende Schichtdicke der Blutgefäße
$\Delta d$     : Pulshub (nur der dynamisch pulsierender Anteil der Blutgefäße)
$\varepsilon$     : Extinktionskoeffizient vom Hämatrokiten bei der zu messenden Wellenlänge

$$a = C_{häm} {}^* \varepsilon$$

$k_{verlust}$ : Verlust durch Gewebeabsorption und Gewebestreuung (Konstante; unbeeinflußt durch Blutpulsation)

$$I_{meß} = I_{in} * e^{-d*a} - k_{verlust} \qquad (14) \text{ Lambert-Beersches Gesetz}$$

daraus folgt für kleine $\Delta d_{Gefäß}$:

$$\frac{\Delta I}{\Delta d} = \frac{\partial I}{\partial d} = -a * I_{meß} \qquad (16)$$

$$\Delta d = - \frac{\Delta I .}{a * I_{meß}}$$

Bildet man das Verhältnis zwischen Kalibrationswert $\Delta d_1/\Delta d_{meß}$ und setzt ihn in die obige Formel ein, so erhält man :

$$C_{meß} = C_1 * \frac{\Delta \alpha_{meß} * \Delta I_1 * I_{meß}}{\Delta \alpha_1 * \Delta I_{meß} * I_1} \qquad (18) \text{ aus 12 und 16}$$

Die Möglichkeit den Pulshub durch die zugehörige Extinktion auszudrücken, fuhrt auf ein weiteres Verfahren, daß ohne Eichmessung auskommt. Es gilt:

$$\Delta \alpha_{meß} = C_{meß} * \alpha_0 * \Delta d_{meß} \qquad (20) \text{ aus 10}$$

$$\Delta d_{meß} = \Delta I_{meß} / (I_{meß} {}^* - a_{Blut}) \qquad (22) \text{ aus 16}$$

$$C_{meß} = \frac{\Delta \alpha * I_{meß} * a_{Blut}}{\alpha_0 * |\Delta I|} \qquad (24) \text{ aus (20) und (22)}$$

und erweitert mit $1/\Delta t$ ergibt sich anschaulich als Verhältnis zweier zeitlicher Steigungen:

$$C = \frac{\dfrac{\Delta\alpha}{\Delta t} * a * I}{\dfrac{|\Delta I|}{\Delta t} * \alpha_0} \Bigg|_{me\beta} \qquad (26) \qquad \text{Blutzucker-}$$

$$\text{konzentration}$$

Praktisch bedeutet es, daß zwei Steigungen gemessen und miteinander ins Verhältnis gesetzt werden. Dabei beschreibt der Ausdruck $\Delta\alpha/\Delta t$ die Tangentensteigung der Polarisation und $\Delta I/\Delta t$ die Tangensteigung der Lichtintensität. Fur die der Ausdruck $\Delta\alpha/\Delta t$ die Tangentensteigung der Polarisation und $\Delta I/\Delta t$ die Tangensteigung der Lichtintensität. Für die Messungen ist ein gemeinsamer Bezugspunkt erforderlich an dem die Steigung bestimmt wird. Hierzu empfiehlt sich der Wendepunkt beim ansteigenden Puls. Verluste durch Streuung, Einkopplung, Verluste im Lichtwellenleiter, usw. sind multiplikativ sowohl in $I_{me\beta}$ als auch in $|\Delta I|$ vorhanden.und kürzen sich somit ebenfalls heraus. In Fig.25 ist dies graphisch dargestellt.

Bei experimentell ermitteltem Faktor $a/\alpha_0$ läßt sich mit Hilfe dieser Formel die Blutzuckerkonzentration mit nur einem Strahler und einem Detektor (und einer Referenz) bestimmen.

Statt der oben beschriebenen Transmissions- Mesung ist auch eine Remissions- Messung möglich . Hierbei könnte das Gerät z.B.: wie eine Armbanduhr getragen werden. Das polarisierte Licht wird hierzu seitwärts in das Gewebe eingestrahlt . Durch Streuung gelangen die Photonen auf ihrer Bahn wieder aus dem Gewebe heraus. Trotz Streuung bleibt die Vorzugsrichtung der Polarisation erhalten. Sie wird nur durch die optisch aktive Substanz (hier: Blutzucker ) gedreht.

Bei der Remissions- Messung ist zu beachten, daß zwei unterschiedliche Wege für die Polarisation und für die Extinktion zu berücksichtigen sind, so daß ein Korrekturfaktor k nötig wird.

Für den mittleren Weg der Extinktion gilt bei Remission: daß sich die ankommenden Photonen im Gewebe aus ihrer Abstrahlrichtung nahezu halbkreisförmig auf den IR- Detektor zu bewegen.

Für den mittleren Weg der Polarisation gilt bei Remission: daß für die Drehung der Schwingungsrichtung der ankommenden Photonen nur die kürzeste Verbindung zwischen Quelle und Sensor im Gewebe zugrunde gelegt werden kann, weil die 'Umwege' sich derart kompensieren, daß z.B.auf dem Hinweg und auf dem Rückweg entgegengesetzte Drehungen stattfinden, so daß diese sich wieder aufheben.

Um bei der Remission den Weg- Korrekturfaktor nahezu zu "1" werden zu lassen, ist die Vorrichtung so aufzubauen, daß die einstrahlende Lichtkeule möglichst nur flach ins Gewebe eintaucht, so daß sich die effektiv wirkenden Wege für die Extinktion und für die Polarisation kaum voneinander unterscheiden. Eine derartige Remissionss- Messung entspricht beinahe einer Transmissions- Messung.

Um Meßungenauigkeiten zu kompensieren, z.B.: durch Verrutschen des Armband- Gerätes, können mehrere gleichartige Detektoren und Sender gleichzeitig angebracht werden. Hierbei werden die Sender einzeln nacheinander eingeschaltet. Die zugehörigen IR-Detektoren messen die ankommenden Intensitäten bzw Polarisationen ( unter Berücksichtigung der vorgegebenen Abstände) gleichzeitig. Das Mehrstrahl- Meßverfahren bewirkt, daß einzelne Unregelmäßigkeiten ( Inhomogenitäten im Gewebe, 'tauber' Sender, 'blinder' Empfänger) kompensiert werden.

Die Vorteile dieser Lösung bestehen darin, daß die zur Bestimmung des Polarisationswinkels erforderliche hohe Genauigkeit hierbei nur noch von der Auflösung des Winkelcodieres/ Winkelgebers, also der Refernz, abhängig ist. Der Vorteil gegenüber zeitlich diskret messenden Systemen (wie z.B. das oben erwähnte US-Patent 5,009,230) liegt darin , daß die Anforderung an die Auswertelektronik sehr viel geringer sein kann. Bei kontinuierlicher, z.B. sinusförmiger Modulation, steckt die gesuchte Information nur in dieser Modulationsfrequenz, so daß alle übrigen Frequenzen herausgefiltert werden können und somit das kleine Signal gezielt aus dem Rauschen heraus verstärkt werden kann.

Durch Modulation des Signals auf eine für die Auswertelektronik günstige Frequenz ergibt sich ein geringeres 1/f - Rauschen der Auswertelektronik (z.B. günstig für Operationsverstärker im Bereich von 100 Hz bis 30 kHz) im Gegensatz zu zeitdiskret messenden Systemen.

Durch diese Kombination der Polarisationsmessung (hier über Phasenmodulation) mit einer Extinktionsmessung können pro Lichtwellenlänge zwei unabhängige Messungen über ein und denselben Sensor vorgenommen werden . Somit wird Material und Abgleicharbeit der Elektronik eingespart.

Die Kombination beider Messungen ermöglicht bereits eine gewebeunabhängige Blutzuckerbestimmung . Deshalb reicht statt einer Transmissionsmessung bereits eine Streumessung (Remissionsmessung) zur Blutzuckerbestimmung aus. Das Verfahren nach dem oben erwähnten US-Patent 5,009,230 arbeitet nur sinnvoll nichtinvasiv bei bekannter

EP 0 674 492 B1

Schichtdicke oder mindestens zwei Meßystemen.

Durch das nichtinvasive Meßverfahren ergibt sich, daß die Messung schmerzlos und nicht infektiös ist. Bei Auftreten kritischer Blutzuckerwerte kann der Diabetiker unmittelbar vom Gerät gewarnt werden. Ein weiterer Vorteil des Verfahrens ist, daß die Blutzuckermessungen auf Dauer erheblich billiger werden als bei täglicher Anwendung von Teststreifen (bis zu 5* 1.50 DM täglich). Insbesondere ältere Diabetiker leiden häufig unter Sehschwächen und können daher mit den herkömmlichen Geräten schlecht hantieren. Durch die beschriebene Meßvorrichtung (z.B. als Armbandgerät) lassen sich daraus resultierende Probleme vermeiden.

In den Zeichnungen ist die Erfindung in verschiedenen Ausführungsformen beispielsweise veranschaulicht. Es zeigen:

Fig.1: schematisch die Anordnung der Meßvorrichtung in der Aufbau- Reihenfolge Lichtquelle, Polarisator, Meßobjekt, priodisch veränderlicher Analysator, Refrenzsignal vom Analysator, optischer Detektor und die elektronische Meßwertauswertung. Neben der Polarisation wird über die Meßelektronik auch die Extinktion ausgewertet, indem möglichst monochromatisches Licht gemessen wird (je monochromatischer das Meßsystem hierzu aufgebaut ist, desto besser wird der Signal/Rauschabstand).

Fig.2: als Alternative einen periodisch sich verändernden Polarisator statt des periodisch sich verändernden Analysators.

Fig.3: als Alternative mit periodisch veränderlichem Analysator, wobei das Referenzsignal vom Kraftfeld direkt zur Auswertelektronik gelangt.

Fig.4: als Alternative einen periodisch sich verändernden Polarisator, wobei auch hier das Referenzsignal direkt vom Kraftfeld bzw vom Rotationsantrieb zur Auswertelektronik gelangt.

Fig.5: als Alternative zwei sich periodisch verändernde Kraftfelder bzw Rotationsantriebe, deren Periode unterschiedlich sein muß (z.B. um 180° phasenverschoben).

Fig.6: die Möglichkeit der Nutzung der Extinktionsmessung bei mehreren monochromatische Licht-Wellenlängen.

Fig.7: die praktische Realisierung der Meßvorrichtung im Querschnitt und in der Draufsicht, z.B. am Finger als Meßobjekt. Unterschiedliche Lichtwellenlängen von polarisiertem Licht können hierbei parallel durch das Meßobjekt und den rotierenden Analysator gesendet werden. Der Analysator besteht aus einer flachen polarisierenden Scheibe. Am Außenrand der Analysatorscheibe wird die momentane Phasenlage über eine Referenzmessung ohne Meßobjekt bestimmt. Diese Phasenreferenzmessung kann optisch, elektrisch oder induktiv vorgenommen werden.

Fig.8: als Alternative zu Fig.7 ist hierbei die Reihenfolge zwischen Meßobjekt und Analysator vertauscht.

Fig.9: ein polarisisationserhaltender Lichtwellenleiter liefert zur optischen Referenzmessung das Licht, so daß eine zusätzliche Lichtquelle eingespart werden kann.

Fig.10; als Alternative zu Fig.9 wird die Reihenfolge von Meßobjekt und Analysator vertauscht.

Fig.11: eine Variante, bei der die Referenzmessung direkt hinter der polarisierenden Scheibe vorgenommen wird, wobei das Licht über einen Lichtwellenleiter zum Referenz-Detektor geleitet wird, bzw dieser Detektor direkt hinter der polarisierenden Scheibe angebracht ist. Der Lichtwellenleiter hinter dem Analysator braucht nicht polarisationserhaltend zu sein. Die Referenzspur ist hierbei die polarisierende Scheibe selbst.

Fig.12: eine Vorrichtung, die eine Streumessung (Remissionsmessung) durch das Gewebe vornimmt. Der optische Detektor befindet sich hierbei bezogen auf das Meßobjekt auf derselben Seite wie die Lichtquelle.

Fig.13 als eine Alternative zur Fig. 12, wobei der optische Detektor und der optische Referenz-Detektor aus ein und derselben Lichtquelle gespeist werden. Der Lichtwellenleiter hierbei muß wieder polarisationserhaltend sein.

Fig.14: polarisationserhaltende Lichtwellenleiter sowohl als Sende-Optode (2 als auch als Empfangs-Optode (2 bei der Transmission. Der Wirkungsgrad der Ein- und Auskopplung kann über Linsensysteme verbessert werden. Mit dieser Figur wird dargestellt, daß mit Hilfe polarisationserhaltender Lichtwellenleiter die Optoden auch z.B. aus sicherheitstechnischen Gründen extern angebracht , d. h. nicht direkt am Meßgerät positioniert sein müssen.

Fig.15: als Alternative zu Fig.14 wird hier eine Remissionsmessung über polarisationserhaltende Lichtwellenleiter gezeigt, wobei die Meßvorrichtung ebenfalls nicht direkt am Meßobjekt positioniert sein muß

Fig.16: ein Armbandgerät, daß der Diabetiker wie eine Armbanduhr ständig bei sich tragen kann. Das Gehäuse (60 beeinhaltet die gesamte Meßvorrichtung mit Batterie. Über ein Armband (67 ist das BlutzuckerMeßgerät fest mit dem Arm verbunden. Auch hier wird eine rotierende polarisierende Scheibe (4 verwendet. Detektor und Quelle sind jeweils autauschbar.

Fig.17: als Alternative zu Fig.16 eine Remissionsmessung (Streumessung) mit dem Armbandgerät. Auch hierbei sind Quelle und optischer Detektor austauschbar.

Fig. 18: als Blockschaltbild die komplette Realisierung des Meßgerätes zusammen mit der zugehörigen Meßelektronik. Zur Bestimmung des Polarisationswinkels wird über einen phasenempfindlichen Detektor das modulierte Licht wieder demoduliert und als Spannungswert gemessen.

Fig.19: zeigt als Alternative zu Fig.20 das Blockschaltbild mit vertauschter Reihenfolge. Hierbei befindet sich das Meßobjekt hinter dem Analysator.

Fig 20: als Blockschaltbild die komplette Realisierung des Meßgerätes zusammen mit der zugehörigen Meßelektronik. Zur Bestimmung des Polarisationswinkels wird der Phasenverlauf durch das Meßobjekt mit dem der Referenzphase verglichen. Hierzu wird der Differenzphasenwinkel zwischen den beiden Nulldurchgängen ermittelt und zur Berechnung der Glukosekonzentzration benutzt.

Fig.21: zeigt als Alternative zu Fig.22 das Blockschaltbild mit vertauschter Reihenfolge. Hierbei befindet sich das Meßobjekt hinter dem Analysator.

Fig.22: die Wandlung des Polarisationswinkels durch den rotierenden Analysator in einen Phasenwinkel. Der mathematische Zusammenhang ist hierbei bildlich dargestellt.

Fig.23: beschreibt den differentiellen Anstieg der Extinktion aufgrund der Blutpulsation, und den synchron dazu sich differentiell verändernde Polarisationswinkel verursacht durch den Blutzucker.

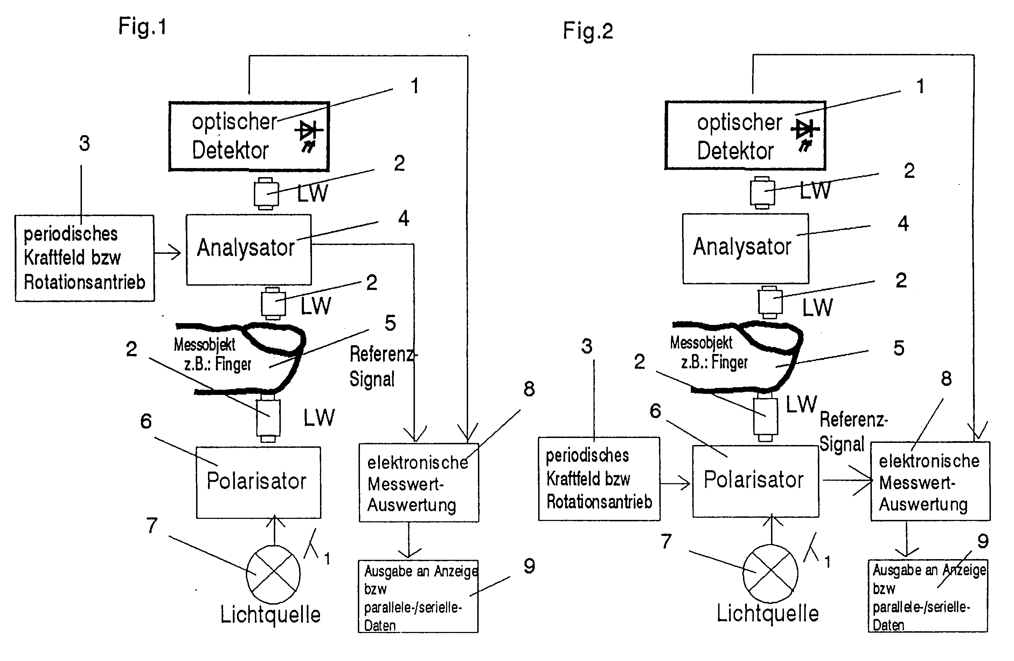

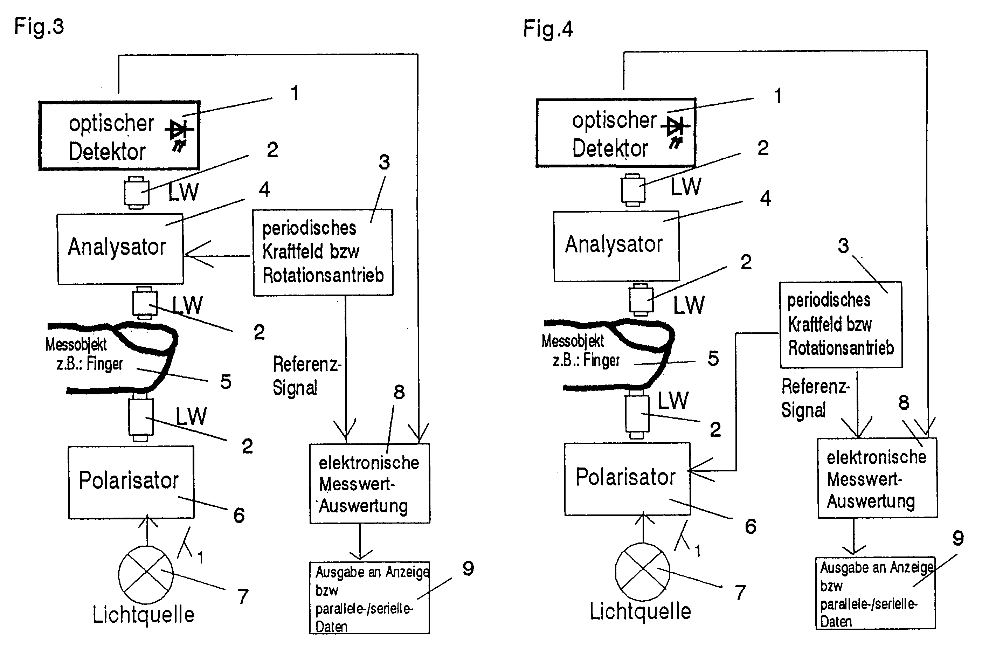

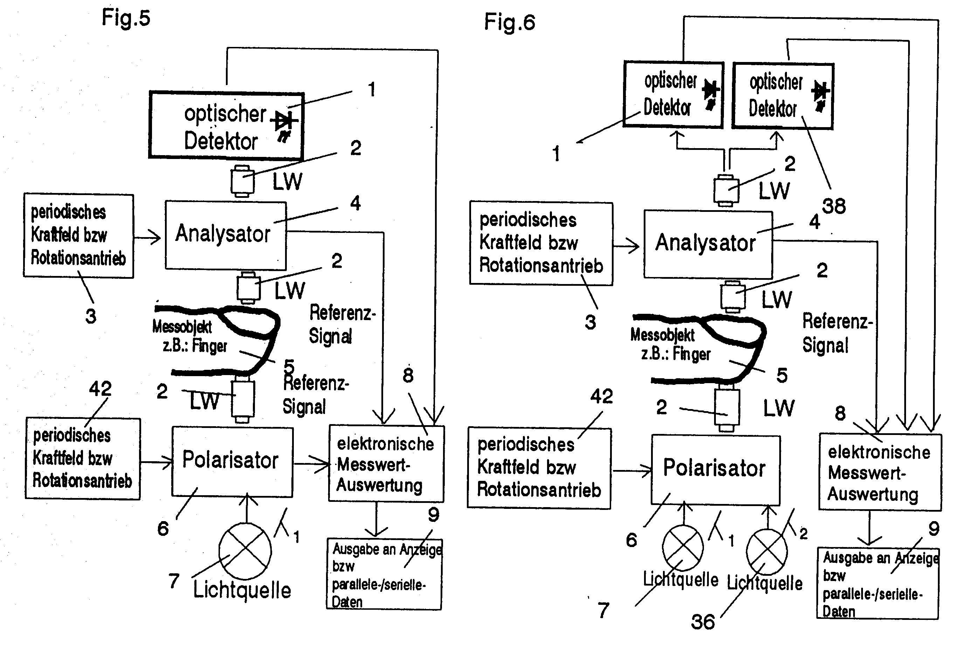

Das in Fig.1 bis Fig.5 veranschaulichte rotierende Polarimeter besteht aus einem optischen Detektor 1 und einer Lichtquelle 7, die über einen Polarisator 6 polarisiertes Licht aussendet. Das polarisierte Licht gelangt über einen polarisationerhaltenden Lichtwellenleiter 2 oder direkt über den Luftweg in das Meßobjekt 5, das hier als Finger dargestellt ist. Über ein periodisches Kraftfeld bzw einem Rotationsantrieb 3 wird ein Analysator 4 periodisch beeinflußt, so daß er kontinuierlich seinen Polarisationswinkel ändert. Der Detektor oder die Lichtquelle 7 sollten einzeln oder zusammen monochromatisches Licht aussenden bzw empfangen können, um gleichzeitig neben der Phasenmessung eine Extinktionsmessung durchführen zu können. Um unabhängig von der Sauerstoffsättigung im Blut zu messen, sind Lichtwellenlängen um 550 nm und um 805 nm besonders geeignet. Auch weitere monochromatische Lichtwellenlängen können mit der Lichtquelle 36, dem Polarisator 37 und dem optischen Detektor 38 parallel ausgewertet werden. Der Lichtwellenleiter 2 muß polarisationserhaltend sein Er kann auch ganz fehlen, so daß das Licht über Luft oder direkt ins Gewebe ein oder ausgekoppelt wird.

Das jeweilig periodisch rotierende System 4 oder 6 liefern ein Referenzsignal an die Meßelektronik, welches nicht vom Meßobjekt 5 beeinflußt wird. Dieses Referenzsignal kann auch direkt vom periodischen Kraftfeld bzw. vom Rotationsantrieb 3 an die Meßelektronik gegeben werden. Von dort wird das Referenzsignal entweder direkt auf eine Anzeige 9,82 bzw auf einen parallelen oder seriellen Datenbus 9,83 gegeben.

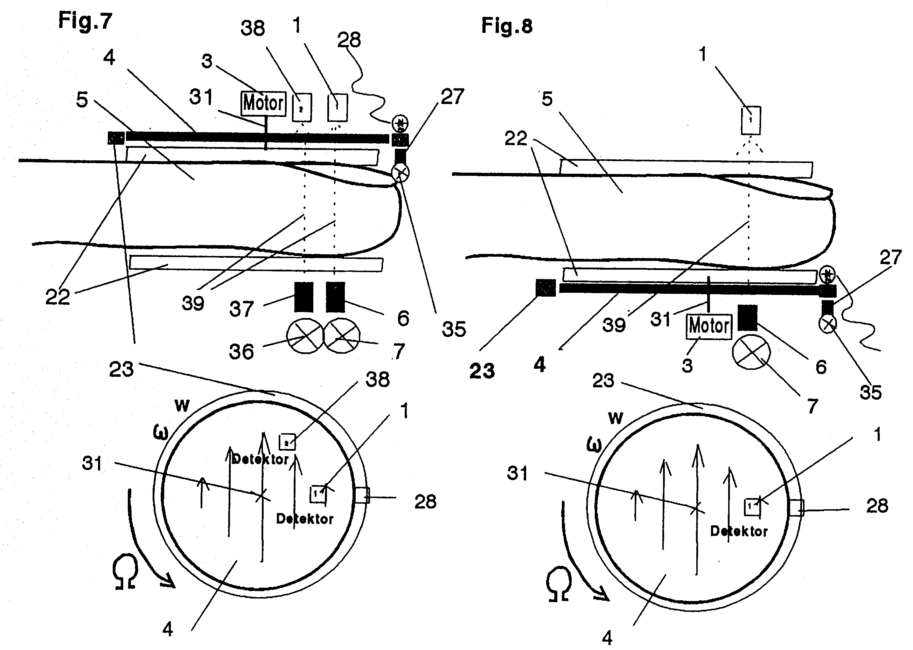

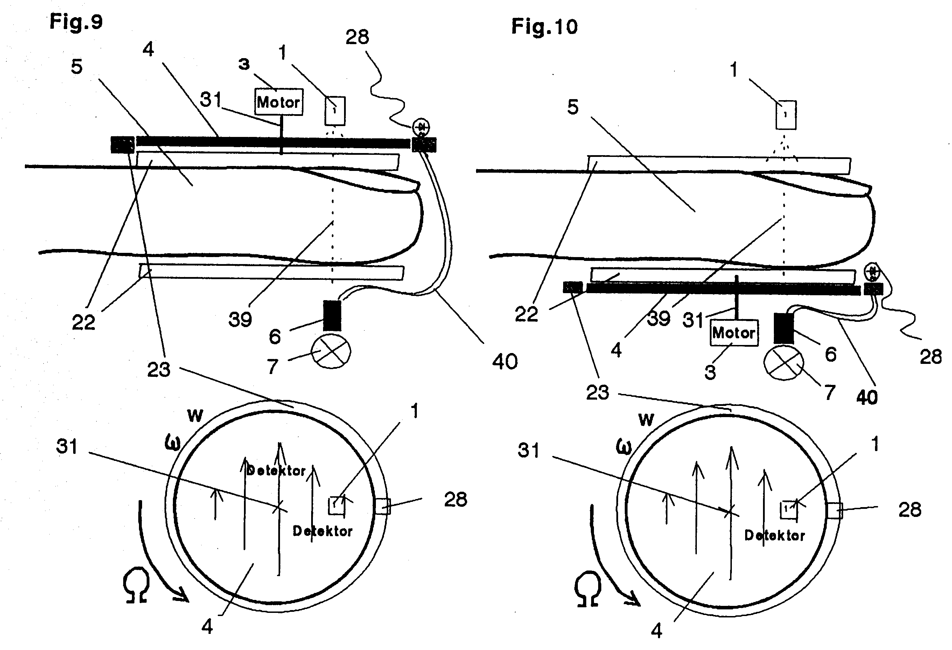

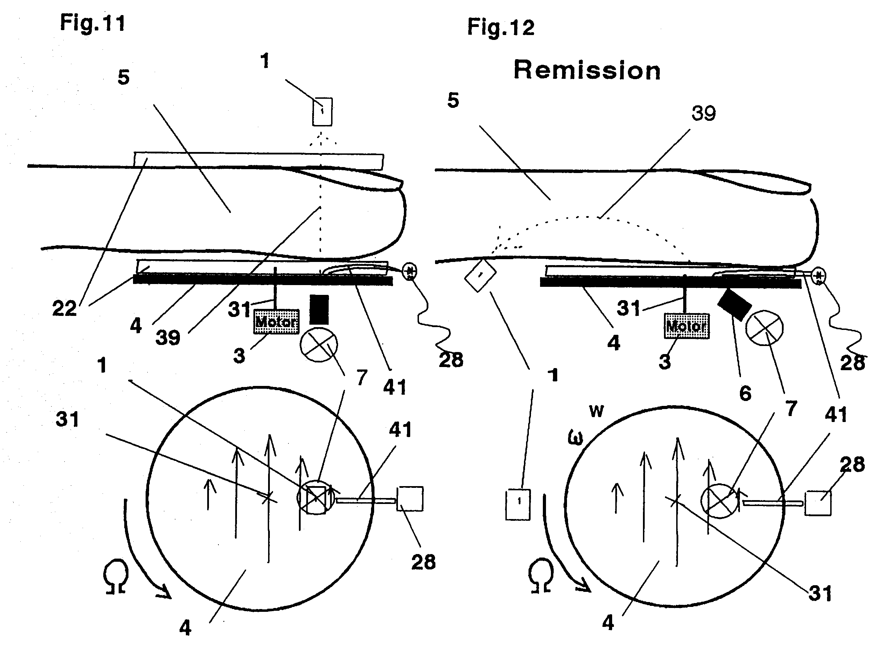

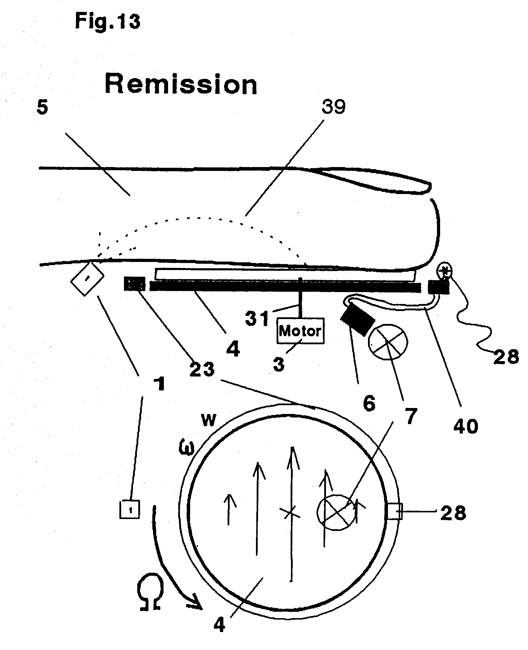

In den Fig.7 bis Fig.13 ist ein praktisches Beispiel dieser Vorrichtung mit einer polarisierenden Analysatorscheibe 4 dargestellt. Die Referenzmessung wird entweder durch den periodisch veränderlichem Polarisator/Analysator selbst vorgenommen oder das Referenzsignal wird z.B. bei einer polarisierenden Scheibe über eine seperate Spur 23 abgenommen. Die Spur besteht entweder aus der modulierenden Scheibe selbst, aus einer Widerstandsschicht, einer ferromagnetischen Schicht, einer reflexiven oder einer lichtdurchlässigen Schicht. Dem vorgesehenen Sensor 28 wird dann ein in Rotationsrichtung periodisches Signal geliefert. wobei die Referenzspur 23 z.B. sinusförmig oder z.B. rechteckig in Rotationsrichtung für den Sensor 28 moduliert ist. Eine durchsichtige mechanische und polarisationserhaltende Halterung 22 trägt dabei die Achse 31, die zugehörige Analysatorscheibe 4 und den Motor bzw Antrieb 3 sowie den übrigen Aufbau. In diesem konkreten Aufbau wird das Referenzsignal über einen optischen Detektor 28 gemessen. Hierbei gelangt das Licht entweder direkt oder über einen polarisationserhaltenden Lichtwellenleiter 40 auf den Photosensor, wobei das polarisierte Referenzlicht nicht direkt von der eigentlichen Lichtquelle 6, sondern auch über eine separate Lichtquelle 35 mit zugehörigem Polarisator 27 erzeugt werden kann. Ein Lichtwellenleiter 41, der nicht irgendwo zwischen Polarisator , Meßobjekt und Analysator angeordnet ist, braucht nicht polarisationserhaltend zu sein.

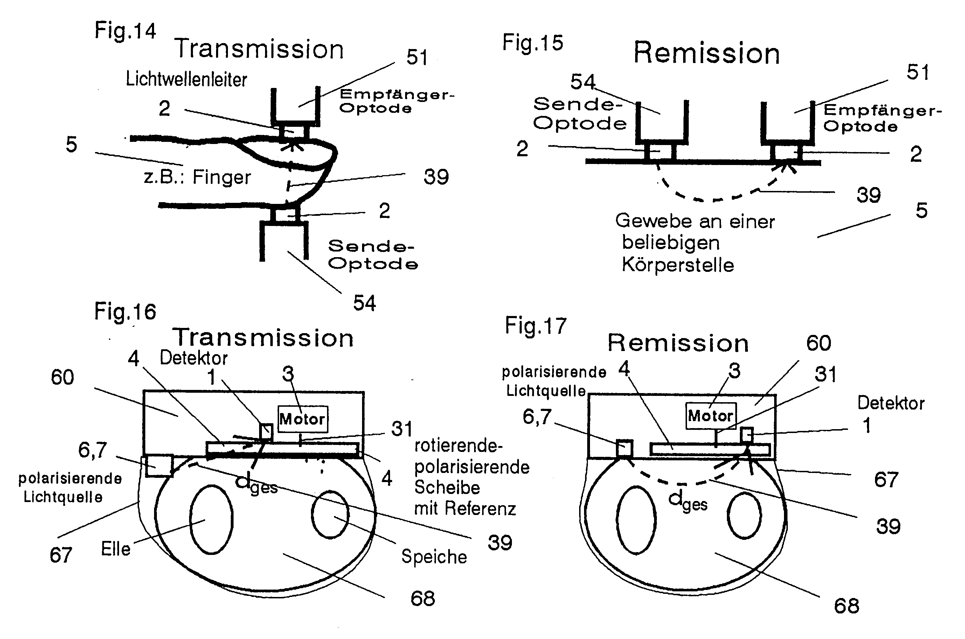

In Fig.14 und Fig.15 wird das Signal über polarisationserhaltende Lichtwellenleiter 2 geleitet. Eine Sendeoptode 54 und eine Empfängeroptode 51 sind gegen Fremdlicht, gegen Störeinflüsse und mechanische Beanspruchung durch eine äußere lichtundurchlässige Hülle geschützt.

In Fig.16 und Fig.17 ist der Aufbau des Gerätes als Armbandgerät dargestellt. Hierbei wird die gesamte Vorrichtung von dem Gehäuse 60 getragen und dieses ist über das Armband 67 am Arm 68 befestigt.

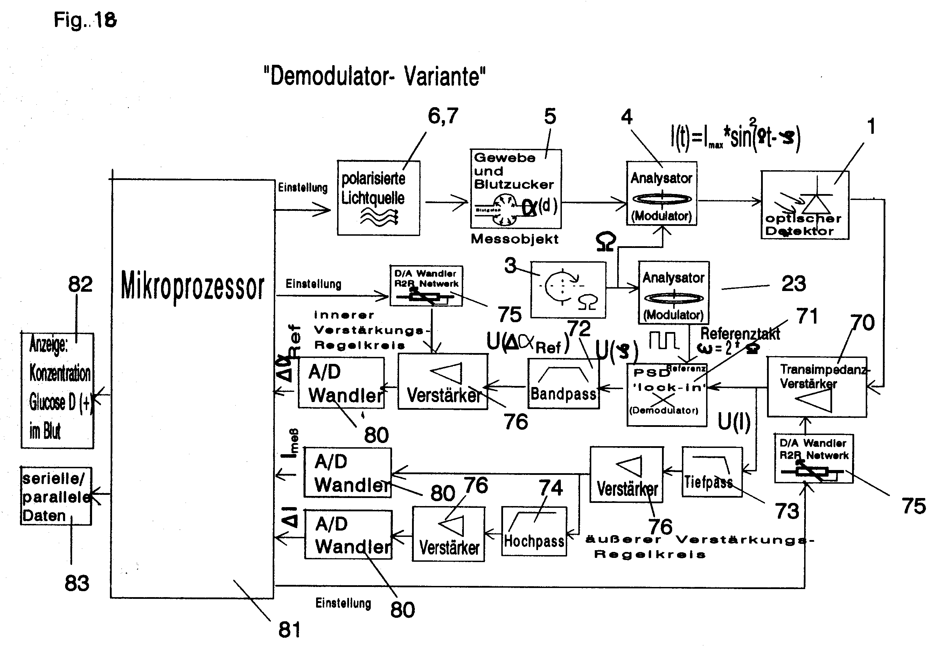

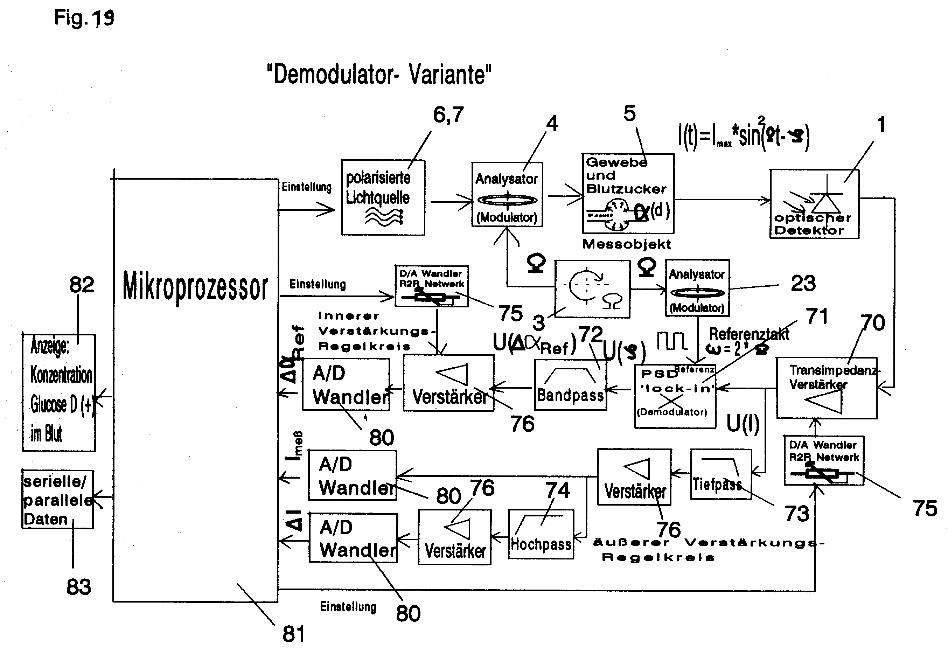

In Fig.18 und Fig.19 wird die elektronische Meßauswertung des Signals beschrieben. Hierbei wird über einen Transimpedanzverstärker 70 der ankommende Strom in eine proportionale Spannung gewandelt. Dieser Wert wird dann parallel über drei Zweige ausgewertet, und die ausgewerteten Ergebnisse werden dem Mikroprozessor 81 als digitale Werte zugeführt. Die drei einzeln aufgeführten Analog/Digital-Wandler 80 stehen exemplarisch für einen gemultiplexten Wandler. In dem ersten Zweig wird der Spannungswert über einen phasensensitiven Detektor (PSD) 71 auf einen Bandpass 72 und von dort auf einen programmierbaren Verstärker geleitet, der hier realisiert ist durch einen Verstärker 76 und einen D/A-Wandler 75. Von dort wird die der Polarisation proportionale Spannung auf den gemultiplexten A/D-Wandler 80 geleitet . Ein paralleler Zweig filtert über einen Tiefpass 73 höherfrequente Signale aus und verstärkt das Signal über einen Verstärker 76. Anschließend spaltet sich dieser Zweig in zwei Zweige auf, wobei der eine direkt auf den gemultiplexten A/D-Wandler 80 gelangt. Der andere Zweig läuft über einen Hochpass 74 und über einen Verstärker 76 auf den gemultiplexten A/D-Wandler.

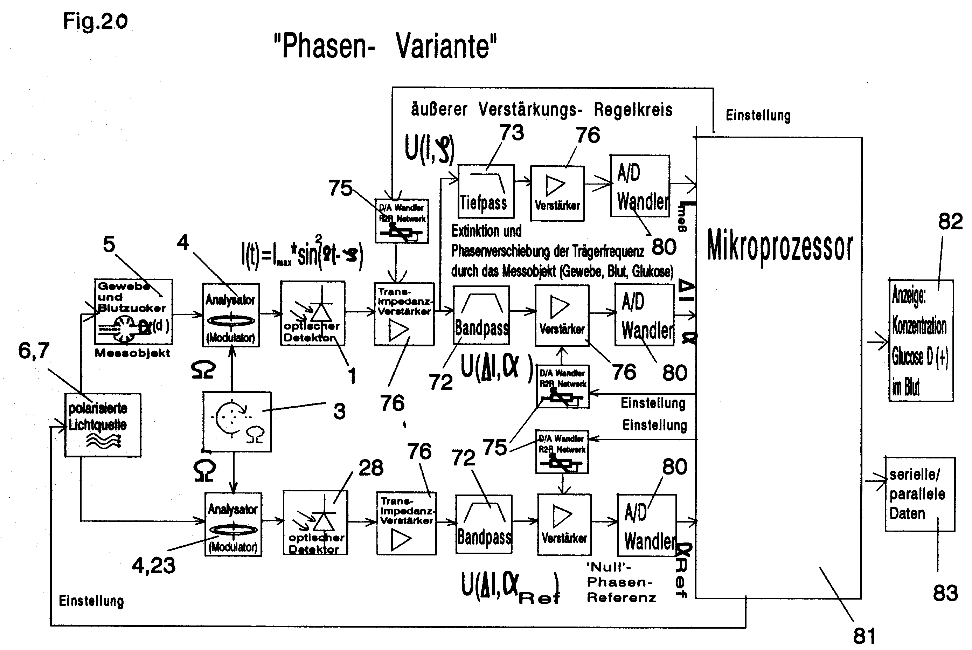

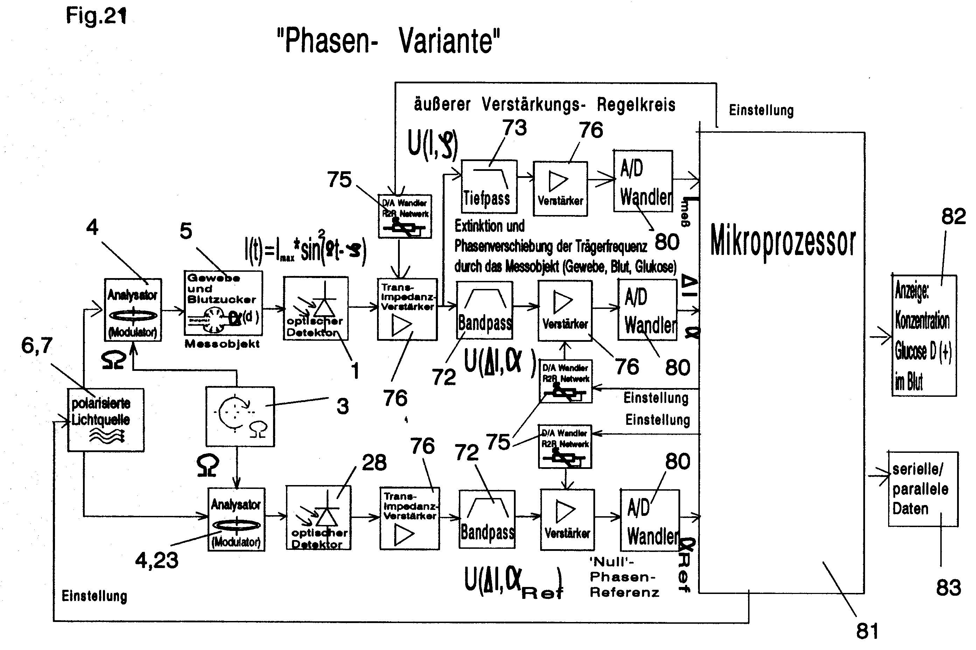

In Fig.20 und Fig.21 wird eine andere Variante der elektronischen Meßauswertung dargestellt. Hierbei finden sich teilweise die vorher genannten Bauteile in einer veränderten Anordnung wieder. Über die Anzeige 9,82 wird der aktuelle Blutzuckerwert angezeigt. Er kann auch als Information in Form von seriellen, parallelen oder analogen Daten zu anderen Empfängern gesendet werden.

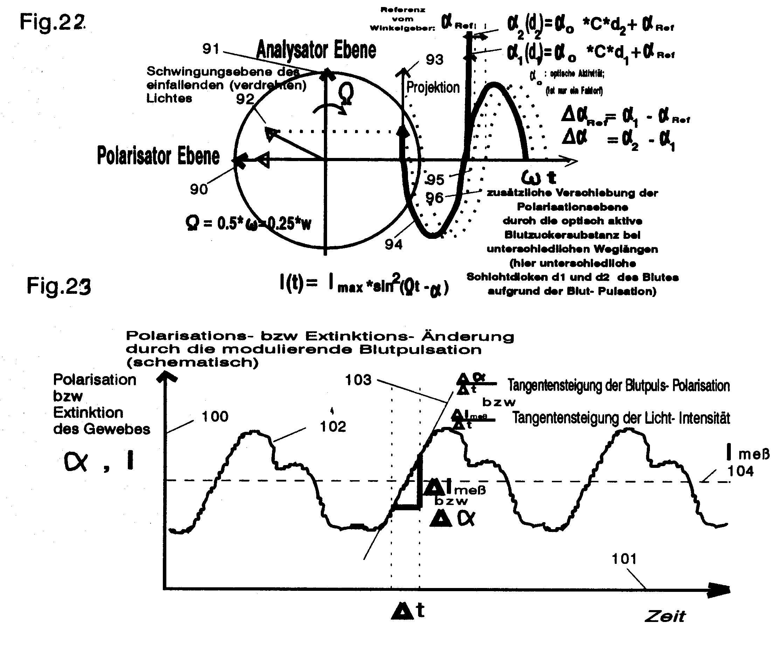

In Fig.22 wird in anschaulicher Weise die Erzeugung der Winkelmodulation der Lichtintensität über die Rotation des Analysators dargestellt. Geht man von einer ursprünglich gekreuzten Stellung von Polarisator-Ebene 90 und Analysator-Ebene 91 aus, so zeigt der mit $\Omega$ rotierende Zeiger 92 die Verschiebung dieser Ebenen zueinander aufgrund der Rotation des Analysators an. Die Projektion dieses Zeigers auf die Projektionsachse 93 zeigt die Modulation der Lichtintensität in Abhängigkeit von der Zeigerstellung

In Fig.23 wird schematisch die elektronische Auswertung der Meßdaten sowohl aus der Extinktion als auch aus der Polarisation über die Achse 100 und über die Zeitachse (t) 101 dargestellt. Die Blutzuckerkonzentration wird hierbei sowohl über den Mittelwert 104 der Intensität $I_{meß}$ als auch über den differentiellen Anstieg 103 der Extinktion 102 und der Polarisation aus der Blutpulsation nach Gleichung [26] bestimmt. Der Intensitätsverlauf 94 (Fig.22) zeigt den gemessenen Referenzsignal-Funktionsverlauf ohne Meßobjekt. Die Funktionsverläufe 95 und 96 zeigen die Phasenverschiebung des modulierten Lichtes aufgrund der Blutpulsation bei unterschiedlichen Zuständen, bedingt durch die sich pulsativ verändernde Blutzuckerkonzentration am Sensor.

Nachstehend sind zwei Meß-Varianten erläutert:

Das polarisierte Licht wird über einen rotierenden Analysator moduliert und durchstrahlt das Meßobjekt. Der rotierende Analysator besteht z.B. aus einer Schicht Polarisationsfolie auf einem durchsichtigen polarisationserhaltendem Trägermaterial. Angetrieben wird der Analysator durch einen Elektromotor. Zum Vergleich der Phasenlage des am Detektor einfallenden Lichtes zur Wirkrichtung der Polarisationsfolie befindet sich auf dem Analysator eine Referenzspur. Die Referenzmessung ermöglicht einen Vergleich der Phasenlage mit Meßobjekt zur Phasenlage ohne Meßobjekt.Der Verlauf der Phasendrehung $\alpha$ wird durch unterschiedliche Schichtdicken des Blutpulsstromes moduliert. Über Extinktionsmessungen (statisch und dynamisch) sowie über Polarisationsmessungen (dynamisch) läßt sich die Glukosekonzentration mit Hilfe der Elektronik gewebeunabhängig mit ein und demselben Sensor gleichzeitig bestimmen. Während die Extinktionsmessung z.B. bei einer Wellenlänge 805 nm relativ einfach zu gestalten ist, ist aufgrund der geforderten Genauigkeit für die Polarisationsmessung das entwickelte Polarimeter mit der zugehörigen Referenzmessung erforderlich. Zur Bestimmung des Drehwinkels $\alpha$ und damit der Zuckerkonzentration C bieten sich nun zwei Wege an:

1.)Demodulation mittels PSD (Phasensensitiver Detektor) ("Demodulator Variante"): Der komplette Meßaufbau ist in Fig.20 und Fig.21 dargestellt. Der dem Transimpedanzverstärker nachgeschaltete Hochpass überträgt die Wechselspannung

$$U = - \frac{U_{max}}{2} \cdot \cos(\omega t - 2\alpha)$$

an einen 'Lock-In'-Verstärker. Durch Demodulation im mit $\omega$ ($\alpha=0$) synchronisierten Phasensensitiven Detektor PSD wird daraus:

$$U_{PSD} = \frac{U max}{\pi} \cdot \sin(2\alpha)$$

Da die auftretenden Winkel sehr klein sind gilt $\sin(2\alpha) \cong 2\alpha$ , also auch:

$$\alpha = \frac{U_{PSD}}{(2 \cdot U_{max})} \cdot \pi$$

Der Wert $U_{max} \sim Imax$ wird diskret gemessen und dem Mikroprozessor zur Berechnung von $\alpha$ zugeführt bzw über einen Regelkreis konstant gehalten. Damit liegt die dem Lichtweg d entsprechende Drehung $\alpha$ als Spannung $U_{PSD}$vor.

Motorschwankungen haben durch den PSD keinen Einfluß auf das Meßergebnis. Die gesuchte Winkelgröße der Glukose- Polarisation liegt hierbei als Spannungswert kontinuierlich vor. Die Werte werden kontinuierlich den A/D- Wandlern zugeführt, so daß sie dem Mikroprozessor jederzeit zur Verfügung stehen. Über die digitalisierten Spannungswerte läßt sich die Blutzuckerkonzentration mit einem kleinem Rechnerprogramm nach der oben erwähnten Formel (6) errechnen. Die notwendigen Extinktionswerte (statisch und dynamisch) liegen nach dem Demodulieren wieder direkt als Meßwert vor.

Die Bestimmung der Glukose-Konzentration geschieht per Rechnerprogramm entsprechend der angeführten Formeln. Hierzu wird sowohl der statische Meßwert als auch die differentiellen Ableitungen der Intensität und des Polarisationswinkels des Blutpulses zu vergleichbaren Zeitpunkten bestimmt (z.B.: Steigung im Wendepunkt des ansteigenden Blut- Pulses)

2.) Messung der Phasenverschiebung ("Phasen- Variante"):

Der komplette Meßaufbau ist in Fig.22 und Fig.23 dargestellt. Hierbei wird die Phasenverschiebungen direkt ausgewertet. Der ankommende Funktionsverlauf U($\alpha$) ist entsprechend der optischen Aktivität des Blutzuckers und des zurückgelegten Weges d dem Verlauf für $\alpha = 0$ gegenüber phasenverschoben. Die Phasenverschiebung wird über die Nulldurchgänge der Funktionsverläufe detektiert. An den Nulldurchgängen kann die Verstärkung extrem hoch gewählt werden, und zwar vorzugsweise rechnergesteuert, so daß die zeitliche Auflösung und damit die Meßgenauigkeit nur noch von der Taktfrequenz der Mikroprozessor-Hardware abhängt. Hierzu wird, definiert durch ein Rechnerprogramm, die Verstärkung dem jeweiligen Abstand der 'Ist-' Spannung vom Nulldurchgang angepaßt (z.B. über Kompandierung). Die Nulldurchgänge der Eingangsfunktionen können so sehr viel genauer zeitlich bestimmt werden. Die absolute Polarisations- Winkelgröße von $\alpha$(d) wird über die Periodendauer und den Abstand zur Referenzphase bestimmt. Der Meßwert U($\alpha$1) ist genüber der 'Null-Phasen- Referenz' U($\alpha$) entsprechend der optischen Aktivität des Blutzuckers phasenverschoben. Der absolute Winkel ergibt sich aus dieser Phasenverschiebung gegenüber der Gesamtperiodendauer. Diese ist proportional der Glukose-Konzentration. Die Phasendifferenz wird über die Nulldurchgänge der Funktionsverläufe indiziert. Hierzu kann die Verstärkung an den Nulldurchgängen beinahe unendlich groß gewählt werden (z.B.: über Kompandierung). Die relativ kleine Winkeldifferenz läßt sich hiermit sehr genau indizieren. Für die zugehörige statische Extinktionsmessung wird z.B.die maximale Intensität ausgewertet. Die Bestimmung der Glukose- Konzentration geschieht per Rechnerprogramm entsprechend der angeführten Formeln. Hierzu wird der statische Meßwert und auch die differentiellen Ableitungen der Intensität und des Polarisationswinkels des Blutpulses zu vergleichbaren Zeitpunkten bestimmt (z.B.: Steigung im Wendepunkt des ansteigenden Blut-Pulses).

**Patentansprüche**

1. Vorrichtung zur nicht-invasiven Konzentrationsbestimmung von polarisierenden Stoffen im menschlichen Körper, insbesondere zur Messung der Blutzuckerkonzentration, mit Hilfe eines Polarimeters mit einer Lichtquelle (7), einem dieser nachgeordneten Polarisator (6), einem sich daran nach einer Messstrecke für ein Messobjekt anschliessenden Analysator (4), wobei die Polarisationsrichtungen von Polarisator und Analysator relativ zueinander periodisch verdrehbar sind, einem dem Polarimeter zugeordneten Winkelgeber zur Ermittlung eines die jeweilige Drehlage der Polarisationsrichtungen von Polarisator und Analysator relativ zueinander ohne Messobjekt in der Messstrecke angebenden periodischen Referenzsignales, einem dem Analysator nachgeordneten Lichtdetektor (1) zur Abgabe eines periodischen Messsignales bei in der Messstrecke vorhandenem Messobjekt und einer elektronischen Auswerteschaltung zur Ermittlung der Konzentration des polarisierenden Stoffes aus dem Mess- und Referenzsignal, dadurch gekennzeichnet, dass die Auswerteschaltung (81) Einrichtungen aufweist, um

   a) die zeitliche Änderung ($\Delta\alpha/\Delta t$) der durch den Blutzuckergehalt verursachten Drehung der Polarisationsebene und
   b) die Lichtintensität (I) nach der Messstrecke sowie deren zeitliche Änderung ($\Delta I/\Delta t$) und daraus die Blutzuckerkonzentration (C) zu bestimmen gemäss

$$C = A \cdot I \cdot \frac{\Delta\alpha/\Delta t}{\Delta I/\Delta t}$$

   wobei A eine experimentiell bestimmte Konstante ist.

2. Vorrichtung nach Anspruch 1, dadurch gekennzeichnet, dass zur periodischen Veränderung der Polarisationsrichtungen von Polarisator und Analysator relativ zueinander Polarisator und/oder Analysator mechanisch drehangetrieben sind.

3. Vorrichtung nach Anspruch 1, dadurch gekennzeichnet, dass zur periodischen Verdrehung der Polarisationsrichtungen von Polarisator und Analysator relativ zueinander die Polarisationsrichtungen von Polarisator und/oder Analysator mit Hilfe eines magnetischen Feldes verdrehbar sind.

4. Vorrichtung nach Anspruch 1, dadurch gekennzeichnet, dass zur periodischen Verdrehung der Polarisationsrichtungen von Polarisator und Analysator relativ zueinander die Polarisationsrichtungen von Polarisator und/oder Analysator mit Hilfe eines elektrischen Feldes verdrehbar sind.

5. Vorrichtung nach einem der vorhergehenden Ansprüche, dadurch gekennzeichnet, dass die Lichtquelle (7) Halbleiter- oder Laserdioden aufweist.

6. Vorrichtung nach einem der vorhergehenden Ansprüchen, dadurch gekennzeichnet, dass zwischen Polarisator (6) und Analysator (4) Lichtwellenleiter (2) vorgesehen sind, und dass der Analysator (4) ebenfalls über einen Lichtwellenleiter (2) mit dem Lichtdetektor (1) verbunden ist.

7. Vorrichtung nach einem der vorhergehenden Ansprüchen, dadurch gekennzeichnet, dass der Analysator (4) eine polarisierende Scheibe aufweist, der eine Spur (23) für die Erzeugung eines Referenzsignales zugeordnet ist.

8. Vorrichtung nach Anspruch 7, dadurch gekennzeichnet, dass die Spur (23) eine Widerstandsschicht, eine ferromagnetische Schicht oder reflexives oder transmittives Material aufweist.

9. Vorrichtung nach einem der vorhergehenden Ansprüchen, dadurch gekennzeichnet, dass Polarisator (6) und Analysator (4) als gegen Fremdlicht geschützte Sendeoptode (54) bzw. Empfängeroptode (51) ausgebildet sind.

10. Vorrichtung nach einem der vorhergehenden Ansprüchen, dadurch gekennzeichnet, dass zur Verbesserung des Signal/Rauschabstandes eine Filtereinrichtung (73, 74) zur Unterdrückung von Störfrequenzen vorgesehen ist.

11. Vorrichtung nach einem der vorhergehenden Ansprüche, dadurch gekennzeichnet, dass die Auswerteschaltung zur Unterdrückung von Drehschwankungen von Polarisator und/oder Analysator einen Demodulator (71) mit Phasenverriegelung aufweist.

12. Vorrichtung nach einem der Ansprüche 1 bis 10, dadurch gekennzeichnet, dass die Auswerteschaltung einen Phasendetektor (72) aufweist.

13. Vorrichtung nach einem der vorhergehenden Ansprüchen, dadurch gekennzeichnet, dass sie als Armbandgerät ausgebildet ist.

14. Verfahren zur nichtinvasiven Bestimmung der Blutzuckerkonzentration, insbesondere mit einer Vorrichtung nach Anspruch 1, dadurch gekennzeichnet, dass folgende Grössen bestimmt werden:

a) die zeitliche Änderung ($\Delta\alpha/\Delta t$) der durch den Blutzuckergehalt verursachten Drehung der Polarisationsebene und
b) die Lichtintensität (I) nach der Messstrecke sowie deren zeitliche Änderung ($\Delta I/\Delta t$),

und dass daraus die Blutzuckerkonzentration (C) bestimmt wird zu

$$C = A \cdot I \cdot \frac{\Delta\alpha/\Delta t}{\Delta I/\Delta t}$$

wobei A eine experimentiell bestimmte Konstante ist.

15. Verfahren nach Anspruch 12, dadurch gekennzeichnet, dass die zeitlichen Änderungen diskontinuierlich, und zwar periodisch mit der Pulsdauer bestimmt werden.

16. Verfahren nach Anspruch 13, dadurch gekennzeichnet, dass die Bestimmung der zeitlichen Änderungen an dem durch den Pulsschlag bestimmten Wendepunkt der Messgrössen erfolgt.

**Claims**

1. Device for non-invasive determination of the concentration of polarising substances in the human body, more particularly for measuring the blood-sugar concentration, with the help of a polarimeter having a light source (7), a polariser (6) arranged downstream thereof, followed by an analyser (4) after a measurement section for a measurement subject, the polarisation directions of the polariser and analyser being adapted to be periodically twisted relative to one another, an angle sensor assigned to the polarimeter for establishing a periodic reference signal indicating the respective rotational position of the polarisation directions of the polariser and analyser relative to one another without a measurement subject in the measurement section, a light detector (1) arranged downstream of the analyser for emitting a periodic measurement signal when there is a measurement subject in the measurement section, and an electronic evaluation circuit for establishing the concentration of the polarising substance from the measurement and reference signal, characterised in that the evaluation circuit (81) incorporates equipment for determining

a) the change over time ($\Delta\alpha/\Delta t$) of the rotation of the polarisation plane caused by the blood-sugar content, and
b) the luminous intensity (I) after the measurement section and the change therein over time ($\Delta I/\Delta t$) and from that the blood-sugar concentration (C) in accordance with

$$C = A \cdot I \cdot \frac{\Delta\alpha/\Delta t}{\Delta I/\Delta t}$$

where A is an empirically determined constant.

2. Device according to claim 1, characterised in that for periodically varying the polarisation directions of the polariser and analyser relative to one another, the polariser and/or analyser are mechanically driven in rotation.

3. Device according to claim 1, characterised in that for periodically twisting the polarisation directions of the polariser and analyser relative to one another, the polarisation directions of the polariser and/or analyser can be twisted with the help of a magnetic field.

4. Device according to claim 1, characterised in that for periodically twisting the polarisation directions of the polariser and analyser relative to one another, the polarisation directions of the polariser and/or analyser can be twisted with the help of an electric field.

5. Device according to any of the preceding claims, characterised in that the light source (7) incorporates semi-conductor diodes or laser diodes.

6. Device according to any of the preceding claims, characterised in that optical waveguides (2) are provided between the polariser (6) and the analyser (4), and that the analyser (4) is likewise connected to the light detector (1) via an optical waveguide (2).

7. Device according to any of the preceding claims, characterised in that the analyser (4) incorporates a polarising disk to which a track (23) is assigned for generating a reference signal.

8. Device according to claim 7, characterised in that the track (23) incorporates a resistor layer, a ferromagnetic layer, or reflective or transmissive material.

9. Device according to any of the preceding claims, characterised in that the polariser (6) and the analyser (4) are respectively in the form of a transmit optode (54) and a receive optode (51) protected against parasitic light.

10. Device according to any of the preceding claims, characterised in that to improve the signal-to-noise interval there is provided a filtering device (73, 74) for suppressing interference frequencies.

11. Device according to any of the preceding claims, characterised in that in order to suppress rotational fluctuations by the polariser and/or analyser the evaluation circuit incorporates a demodulator (71) with phase locking.

12. Device according to any of claims 1 to 10, characterised in that the evaluation circuit incorporates a phase detector (72).

13. Device according to any of the preceding claims, characterised in that it is in the form of a bracelet instrument.

**14.** Method for the non-invasive determination of blood-sugar concentration, more particularly using a device according to claim 1, characterised in that the following variables are determined:

a) the change over time ($\Delta\alpha/\Delta t$) of the rotation of the polarisation plane caused by the blood-sugar content, and
b) the luminous intensity (I) after the measurement section and the change therein over time ($\Delta I/\Delta t$),

and that from this the blood-sugar concentration (C) is determined to be

$$C = A \cdot I \cdot \frac{\Delta\alpha/\Delta t}{\Delta I/\Delta t}$$

where A is an empirically determined constant.

**15.** Method according to claim 12, characterised in that the changes over time are determined discontinuously, notably periodically with the pulse duration.

**16.** Method according claim 13, characterised in that the determination of the changes over time takes place at the turning point of the measurement variables, as determined by the pulsation.

**Revendications**

**1.** Dispositif pour déterminer de manière non-invasive la concentration en substances polarisantes dans le corps humain, notamment pour mesurer la concentration de sucre dans le sang, à l'aide d'un polarimètre comportant une source de lumière (7), d'un polariseur (6), d'un analyseur (4) disposé après une zone de mesure pour un objet à mesurer, les directions de polarisation du polariseur et de l'analyseur pouvant être tournées l'une par rapport à l'autre périodiquement, d'un indicateur d'angle associé au polarimètre pour fournir un signal de référence périodique indiquant la position en rotation des directions de polarisation l'une par rapport à l'autre du polariseur et de l'analyseur sans objet à mesurer dans la zone de mesure, d'un détecteur de lumière (1) disposé après l'analyseur pour fournir un signal de mesure périodique lors de la présence d'un objet à mesurer dans la zone de mesure, et d'un circuit d'évaluation électronique pour fournir la concentration de la substance polarisante à partir des signaux de mesure et de référence, caractérisé en ce que le circuit d'évaluation (81) comporte des dispositifs pour établir

a) la variation dans le temps ($\Delta\alpha/\Delta t$) de la rotation du plan de polarisation provoquée par le taux de sucre dans le sang et
b) l'intensité lumineuse (I) après la zone de mesure ainsi que sa variation dans le temps ($\Delta I/\Delta t$) et ensuite déterminer le taux de sucre dans le sang (C) selon

$$C = A \cdot I \cdot (\Delta\alpha/\Delta t)/(\Delta I/\Delta t)$$

où A est une constante déterminée expérimentalement.

**2.** Dispositif selon la revendication 1, caractérisé en ce que le polariseur et/ou l'analyseur sont entraînés en rotation de manière mécanique pour modifier de manière périodique les directions de polarisation du polariseur et de l'analyseur l'une par rapport à l'autre.

**3.** Dispositif selon la revendication 1, caractérisé en ce que les directions de polarisation du polariseur et/ou de l'analyseur sont mises en rotation à l'aide d'un champ magnétique pour tourner périodiquement les directions de polarisation du polariseur et de l'analyseur l'une par rapport à l'autre.

**4.** Dispositif selon la revendication 1, caractérisé en ce que les directions de polarisation du polariseur et/ou de l'analyseur sont mises en rotation à l'aide d'un champ électrique pour tourner périodiquement les directions de polarisation du polariseur et de l'analyseur l'une par rapport à l'autre.

**5.** Dispositif selon l'une quelconque des revendications précédentes, caractérisé en ce que la source de lumière (7) comporte des diodes semiconductrices ou laser.

**6.** Dispositif selon l'une quelconque des revendications précédentes, caractérisé en ce que des guides d'ondes lumineuses (2) sont disposés entre le polariseur (6) et l'analyseur (4) et en ce que l'analyseur (4) est également relié par un guide d'ondes lumineuses (2) au détecteur de lumière (1).

7. Dispositif selon l'une quelconque des revendications précédentes, caractérisé en ce que l'analyseur (4) comporte un disque polarisant qui est associé à une piste (23) pour établir le signal de référence.

8. Dispositif selon la revendication 7, caractérisé en ce que la piste (23) comporte une couche résistive, une couche ferromagnétique ou un matériau réfléchissant ou transmissif.

9. Dispositif selon l'une quelconque des revendications précédentes, caractérisé en ce que le polariseur (6) et l'analyseur (4) sont configurés en optodes d'émission (54) et de réception (51) protégés contre la lumière étrangère.

10. Dispositif selon l'une quelconque des revendications précédentes, caractérisé en ce qu'il comprend un dispositif de filtrage (73, 74) pour atténuer les fréquences parasites afin d'améliorer le rapport signal/bruit.

11. Dispositif selon l'une quelconque des revendications précédentes, caractérisé en ce que le circuit d'évaluation comprend un démodulateur (71) à verrouillage de phase pour atténuer des fluctuations de rotation du polariseur et/ou de l'analyseur.

12. Dispositif selon l'une quelconque des revendications 1 à 10, caractérisé en ce que le circuit d'évaluation comprend un détecteur de phase (72).

13. Dispositif selon l'une quelconque des revendications précédentes, caractérisé en ce qu'il est sous la forme d'un brassard.

14. Procédé pour la détermination non-invasive de la concentration de sucre dans le sang, notamment à l'aide d'un dispositif selon la revendication 1, caractérisé en ce que l'on détermine les valeurs suivantes :

a) la variation dans le temps ($\Delta\alpha/\Delta t$) de la rotation du plan de polarisation provoquée par le taux de sucre dans le sang et
b) l'intensité lumineuse (I) après la zone de mesure ainsi que sa variation dans le temps ($\Delta I/\Delta t$),

et en ce que l'on détermine à partir de ces valeurs la concentration de sucre dans le sang (C) selon

$$C = A \cdot I \cdot (\Delta\alpha/\Delta t)/(\Delta I/\Delta t)$$

où A est une constante déterminée expérimentalement.

15. Procédé selon la revendication 12, caractérisé en ce que les variations dans le temps sont déterminées de manière discontinue, notamment périodique en fonction de la période du pouls.

16. Procédé selon la revendication 13, caractérisé en ce que la détermination des variations dans le temps est effectuée à partir du point d'inflexion des valeurs de mesure, déterminé par le battement du pouls.

Fig.1

Fig.2

EP 0 674 492 B1

Fig.4

optischer Detektor — 1

LW — 2

Analysator — 4

LW — 2

Messobjekt z.B.: Finger — 5

LW — 2

Polarisator — 6

Lichtquelle — 7

periodisches Kraftfeld bzw Rotationsantrieb — 3

8

Referenz-Signal

elektronische Messwert-Auswertung

Ausgabe an Anzeige bzw parallele-/serielle-Daten — 9

Fig.3

optischer Detektor — 1

LW — 2

Analysator — 4

LW — 2

Messobjekt z.B.: Finger — 5

LW — 2

Polarisator — 6

Lichtquelle — 7

periodisches Kraftfeld bzw Rotationsantrieb — 3

8

Referenz-Signal

elektronische Messwert-Auswertung

Ausgabe an Anzeige bzw parallele-/serielle-Daten — 9

16

Fig.6

optischer Detektor

optischer Detektor

38

LW 2

Analysator 4

LW 2

Messobjekt z.B.: Finger

Referenz-Signal

5 LW

Polarisator

2

Lichtquelle Lichtquelle

36

6

7

1

periodisches Kraftfeld bzw Rotationsantrieb 3

periodisches Kraftfeld bzw Rotationsantrieb 42

elektronische Messwert-Auswertung 8

9 Ausgabe an Anzeige bzw parallele-/serielle-Daten

Fig.5

optischer Detektor 1

LW 2

Analysator 4

LW 2

Referenz-Signal

Messobjekt z.B.: Finger

5 Referenz-Signal

LW 2

Polarisator

Lichtquelle 1

6

7

periodisches Kraftfeld bzw Rotationsantrieb 3

periodisches Kraftfeld bzw Rotationsantrieb 42

elektronische Messwert-Auswertung 8

9 Ausgabe an Anzeige bzw parallele-/serielle-Daten

17

# Fig.7

# Fig.8

EP 0 674 492 B1

**Fig.9**

Motor

Detektor

Detektor

w
ω
Ω

**Fig.10**

Motor

Detektor

w
ω
Ω

EP 0 674 492 B1

Fig.11

Fig.12

Remission

**Fig.13**

# Remission

EP 0 674 492 B1

Fig.14

## Transmission

Lichtwellenleiter

51

Empfänger-Optode

2

5

z.B.: Finger

39

2

Sende-Optode

54

Fig.15

## Remission

54

Sende-Optode

51

Empfänger-Optode

2

2

39

5

Gewebe an einer
beliebigen
Körperstelle

Fig.16

## Transmission

60

Detektor

4

1

3

Motor

31

6,7

$d_{ges}$

rotierende-polarisierende
Scheibe
mit Referenz

polarisierende
Lichtquelle

4

67

Elle

Speiche

39

68

Fig.17

## Remission

60

polarisierende
Lichtquelle

4

3

31

6,7

Motor

Detektor

1

$d_{ges}$

67

39

68

EP 0 674 492 B1

Fig. 18

## "Demodulator- Variante"

$$I(t) = I_{max} * sin^2(\Omega t - \vartheta)$$

polarisierte Lichtquelle **6,7**

Gewebe und Blutzucker $\alpha(d)$ **5**
Messobjekt

Analysator (Modulator) **4**

optischer Detektor **1**

Mikroprozessor

Einstellung

Einstellung

D/A Wandler R2R Network **75**

**3** $\Omega$

Analysator (Modulator) **23**

Referenztakt $\omega = 2 *$ **71**

Transimpedanz-Verstärker **70**

**82** Anzeige: Konzentration Glucose D (+) im Blut

Innerer Verstärkungs-Regelkreis

$\Delta\alpha_{Ref}$

A/D Wandler **80**

Verstärker

$U(\Delta\alpha_{Ref})$ **72** $U(\vartheta)$

Bandpass **76**

PSD 'lock-in' (Demodulator) Referenz

$U(I)$

**23**

serielle/ parallele Daten

$I_{meß}$

A/D Wandler

**80** **76** **74** Verstärker

Tiefpass

D/A Wandler R2R Network **73**

**75**

**83**

$\Delta I$

A/D Wandler **80**

Verstärker **76**

Hochpass

äußerer Verstärkungs-Regelkreis

Einstellung **80**

**81**

EP 0 674 492 B1

Fig. 19

## "Demodulator- Variante"

$$I(t) = I_{max} * \sin^2(\Omega t - \vartheta)$$

**Fig.20**

## "Phasen- Variante"

äußerer Verstärkungs- Regelkreis

Einstellung

$U(I,\vartheta)$

73    76

Tiefpass    Verstärker    A/D Wandler

75    D/A Wandler R2R Network    80    $I_{meB}$

Extinktion und Phasenverschiebung der Trägerfrequenz 80 durch das Messobjekt (Gewebe, Blut, Glukose)

**Mikroprozessor**

82

Anzeige: Konzentration Glucose D (+) im Blut

5    4    $I(t)=I_{max}*sin^2(\Omega t - \vartheta)$

Gewebe und Blutzucker    Analysator (Modulator)    optischer Detektor    Trans-Impedanz-Verstärker    Bandpass    Verstärker    A/D Wandler    $\Delta I$    $\alpha$

$\alpha(d)$    Messobjekt    $\Omega$    1    72    $U(\Delta I,\alpha)$    D/A Wandler R2R Network    76    80

Einstellung

Einstellung

6,7    3    76    75    D/A Wandler R2R Network    80

polarisierte Lichtquelle    $\Omega$    28    76    72

$\alpha_{Ref}$

4,23    Analysator (Modulator)    optischer Detektor    Trans-Impedanz-Verstärker    Bandpass    Verstärker    A/D Wandler

Einstellung    $U(\Delta I,\alpha_{Ref})$    'Null'-Phasen-Referenz

serielle/ parallele Daten

83

81

EP 0 674 492 B1

# Fig.21

## "Phasen- Variante"

**äußerer Verstärkungs- Regelkreis**

Einstellung

$U(l, \mathcal{S})$

75 · D/A Wandler R2R Netwerk

73 · Tiefpass · 76 · Verstärker · A/D Wandler · 80 · $l_{meB}$

Extinktion und Phasenverschiebung der Trägerfrequenz durch das Messobjekt (Gewebe, Blut, Glukose)

**Mikroprozessor**

4 · Analysator (Modulator)

5 · Gewebe und Blutzucker · Messobjekt · $\alpha(d)$

$l(t) = l_{max} * sin^2(\Omega t - \mathcal{S})$

optischer Detektor · 1

Trans-Impedanz-Verstärker · 76

72 · Bandpass · $U(\Delta l, \alpha)$

76 · Verstärker · A/D Wandler · 80 · $\Delta l$ · $\alpha$

D/A Wandler R2R Netwerk

Einstellung

6,7 · polarisierte Lichtquelle

$\Omega$

3

$\Omega$

Einstellung

82 · Anzeige: Konzentration Glucose D (+) im Blut

4,23 · Analysator (Modulator)

28 · optischer Detektor

Trans-Impedanz-Verstärker · 76

72 · Bandpass

76 · Verstärker · A/D Wandler · 80

75 · D/A Wandler R2R Netwerk

$U(\Delta l, \alpha_{Ref})$

'Null'-Phasen-Referenz · $\alpha_{Ref}$

Einstellung

81

serielle/ parallele Daten

83

EP 0 674 492 B1

**Fig.22**

91 — Analysator Ebene

Schwingungsebene des einfallenden (verdrehten) Lichtes 92

Polarisator Ebene

90

$\Omega = 0.5 * \omega = 0.25 * w$

93 — Projektion

Referenz vom Winkelgeber: $\alpha_{Ref}$

$\alpha_2(d_2) = \alpha_0 * C * d_2 + \alpha_{Ref}$
$\alpha_1(d_1) = \alpha_0 * C * d_1 + \alpha_{Ref}$

$\alpha_0$: optische Aktivität; (ist nur ein Faktor)

$\Delta\alpha_{Ref} = \alpha_1 - \alpha_{Ref}$
$\Delta\alpha = \alpha_2 - \alpha_1$

95
96

$\omega t$

zusätzliche Verschiebung der Polarisationsebene durch die optisch aktive Blutzuckersubstanz bei unterschiedlichen Weglängen (hier unterschiedliche Schichtdicken d1 und d2 des Blutes aufgrund der Blut- Pulsation)

94

$$I(t) = I_{max} * \sin^2(\Omega t - \alpha)$$

**Fig.23**

Polarisations- bzw Extinktions- Änderung durch die modulierende Blutpulsation (schematisch)

Polarisation bzw Extinktion des Gewebes

$\alpha$ , I

100

102

103

$\dfrac{\Delta\alpha}{\Delta t}$ Tangentensteigung der Blutpuls- Polarisation

bzw

$\dfrac{\Delta I_{meß}}{\Delta t}$ Tangentensteigung der Licht- Intensität

$\Delta I_{meß}$ bzw $\Delta\alpha$

$I_{meß}$
104

101

$\Delta t$

Zeit

EP 0 674 492 B1